# EUROPEAN PATENT SPECIFICATION

(11) **EP 1 667 719 B1**
(45) Date of publication and mention of the grant of the patent: **24.11.2010**
(21) Application number: 04765359.7
(22) Date of filing: 17.09.2004
(51) Int. Cl.: A61K 45/06, A61P 35/00, A61K 31/497, A61K 31/553

(54) **TREATMENT OF GASTROINTESTINAL STROMAL TUMORS WITH IMATINIB AND MIDOSTAURIN**
BEHANDLUNG VON GASTROINTESTINALEN STROMATUMOREN MIT IMATINIB UND MIDOSTAURIN
TRAITEMENT DE TUMEURS DE STROMA DU TUBE DIGESTIF AVEC L'IMATINIBE ET LA MIDOSTAURINE

(30) Priority: 19.09.2003 US 504245 P
(43) Date of publication of application: 14.06.2006
(73) Proprietor: Novartis AG, 4056 Basel (CH); Novartis Pharma GmbH, 1230 Wien (AT)
(72) Inventor: DIMITRIJEVIC, Sasa, F-68440 Habsheim (FR); FLETCHER, Jonathan, A., Brookline, MA 02446 (US); SILBERMAN, Sandra, Leta, Randolph, NJ 07869 (US)
(74) Representative: Roth, Peter Richard
(86) International application number: PCT/EP2004/010467
(87) International publication number: WO 2005/027971

(56) References cited:
- WO-A-01/47507
- WO-A-02/34727
- WO-A-99/03854
- WO-A-03/072159
- WO-A-03/084543
- COOLS ET AL: "PKC412 overcomes resistance to imatinib in a murine model of FIP1L1-PDGFR-alpha-induced myeloproliferative disease" CANCER CELL, vol. 3, no. 5, 2003, pages 459-469, XP008039395
- SHAH ET AL: "Multiple BCR-ABL kinase domain mutations confer polyclonal resistance to the tyrosine kinase inhibitor imatinib (STI571) in chronic phase and blast crisis chronic myeloid leukemia" CANCER CELL, vol. 2, 2002, pages 117-125, XP009005144
- DANCEY AND SAUSVILLE: "Issues and progress with protein kinase inhibitors for cancer treatment" NATURE REVIEWS, vol. 2, April 2003 (2003-04), pages 296-313, XP008039406

## Description

The invention relates to a combination therapy including imatinib, or a pharmaceutically acceptable salt thereof, and a protein kinase C inhibitor, viz. the staurosporine derivative midostaurin, also known as PKC412, or a pharmaceutically acceptable salt thereof, for the manufacture of pharmaceutical compositions for the treatment of gastrointestinal stromal tumours (GIST), to the use of this combination therapy in the treatment of GIST, and to a treating warm-blooded animals, including humans, suffering from GIST by administering to a said animal in need of such treatment an effective combination of imatinib, or a pharmaceutically acceptable salt thereof, and midostaurin, of a pharmaceutically acceptable salt thereof.

Gastrointestinal stromal tumours (GISTs) are a recently characterized family of mesenchymal neoplasms, which originate from the gastrointestinal tract, most commonly from the stomach (60 to 70% of all GISTs), and also from the esophagus, small intestine, colon and rectum. GISTs can also arise, infrequently, from sides outside the gastrointestinal tract. In the past, these tumours were variously classified as leiomyoma, leiomyoblastoma, or leiomyosarcoma or other types of sarcoma. However, it is now clear that GISTs represent a distinct clinicopathologic set of diseases based on their unique molecular pathogenesis and clinical features. GISTs can be now diagnosed unequivocally - and distinguished from other types of mesenchymal tumors, e.g. by demonstration of typical histological features and/or immunohistochemical evidence for KIT protein expression.

While relatively rare at an estimated incidence of about 20 cases/million, GIST is the most common mesenchymal neoplasm of the gastrointestinal tract. Until recently, the only effective therapy has been surgical resection. The limited value of conventional cytotoxic chemotherapy and radiation therapy has resulted in advanced GIST being an invariably progressive and fatal condition, the median survival of patients varying from 20 months (metastatic GIST) to a year or less (post-surgical recurrence)

Imatinib is a small molecule selectively inhibiting specific tyrosine kinases that has emerged recently as a valuable treatment for patients with advanced GIST. The use of imatinib as monotherapy for the treatment of GIST has been described in PCT publication WO 02/34727. However, it has been reported that primary resistance to imatinib is present in a population of patients, for example 13.7% of patients in one study. In addition, a number of patients acquire resistance to treatment with imatinib. More generally this resistance is partial with progression in some lesions, but continuing disease control in other lesions. Hence, these patients remain on imatinib treatment but with a clear need for additional or alternative therapy.

Protein kinase C is one of the key enzymes in cellular signal transduction pathways, and it has a pivotal role in the control of cell proliferation and differentiation. PKC is a family of serine/threonine kinases. At least 12 isoforms of PKC h ave b een identified, and they are commonly divided into three g roups based on their structure and substrate requirements. PKC expression has been found to be elevated in human breast tumor biopsies as compared with normal breast tissues, and high PKC expression has been considered as a biological marker for malignancy in human astrocytomas. One of the PKC isoforms, PKCθ, is a positive regulator of survival signaling in T cells. Interestingly, PKCθ is activated in GISTs, as manifested by constitutive phosphorylation of PKCθ in GIST and as evidenced by inhibition of PKCθ activity resulting in GIST cell death. Thus, PKCθ may be considered a potential target kinase for therapeutic interventions in GIST. In particular, PKC inhibitors are beneficial in the treatment of imatinib resistant GISTs.

Accordingly, the present invention relates to a treatment of GIST, which comprises administering a combination of imatinib and an inhibitor of protein kinase C, viz. midostaurin, to a patient with GIST.

Imatinib is 4-(4-methylpiperazin-1-ylmethyl)-N-[4-methyl-3-(4-pyridin-3-yl)pyrimidin-2-ylamino)phenyl]-benzamide having the formula I

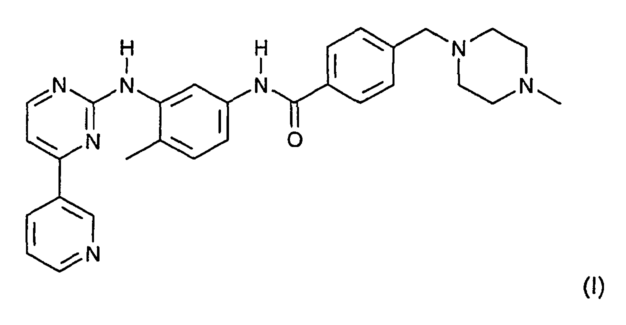

The preparation of imatinib and the use thereof, especially as an anti-tumour agent, are described in Example 21 of European patent-application EP-A-0 564 409, which was published on 6 October 1993, and in equivalent applications and patents in numerous other countries, e.g. in US patent 5,521,184 and in Japanese patent 2706682.

Pharmaceutically acceptable salts are pharmaceutically acceptable acid addition salts, like for example with inorganic acids, such as hydrochloric acid, sulfuric acid or a phosphoric acid, or with suitable organic carboxylic or sulfonic acids, for example aliphatic mono- or dicarboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid.

The monomethanesulfonic acid addition salt of imatinib and a preferred crystal form thereof are described in PCT patent application WO99/03854 published on January 28, 1999. The monomethanesulfonic acid salt of imatinib is marketed in many countries under brandname Glivec^{®} or Gleevec^{™}.

Depending on species, age, individual condition, mode of administration, and the clinical picture in question, effective doses of imatinib, particularly as its monomethanesulfonic acid salt, for example daily oral doses of about 100-1000 mg, preferably 200-600 mg, especially 400 mg, are administered to warm-blooded animals, particularly patient of the species *Homo sapiens,* of about 70 kg bodyweight. For adult human patients with unresectable and/or metastatic malignant GIST, a starting oral dose of imatinib of 400-600 mg daily, particularly 600 mg daily, is recommended according to the present invention. For patients with an inadequate response after an assessment of response to therapy dose escalation of imatinib to 800 or 1000 mg daily can be safely considered and patients may be treated as long as they benefit from treatment and in the absence of limiting toxicities.

Protein kinase C inhibitors and their administration are described in U.S. Patent No. 5,093,330. Midostaurin is a particularly important protein kinase C inhibitor.

Midostaurin, a staurosporine derivative of the formula *N*-[(9*S*,10*R*,11*R*,13*R*)-2,3,10,11,12,13-hexahydro-10-methoxy-9-methyl-1-oxo-9,13-epoxy-1*H*,9*H*-diindolo[1,2,3-gh:3',2',1'-Im]pyrrolo[3,4-j][1,7]benzodiazonin-11-yl]-*N*-methylbenzamide: 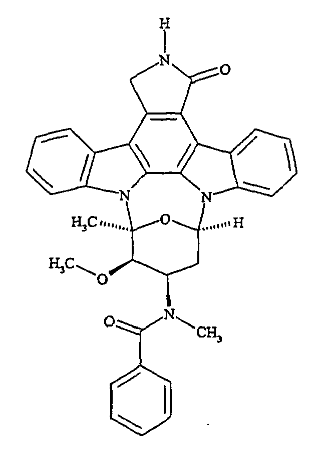 or a salt thereof, is referred as midostaurin or PKC412.

The preparation of midostaurin and its use as selective inhibitor of protein kinase C and the pharmaceutically acceptable salts thereof are described in U.S. Patent No. 5,093,330.

Midostaurin can be administered orally in dosages up to about 300 mg/day, for example 100 to 300 mg/day. The midostaurin is administered as a single dose or split into two or three doses daily, preferably two doses. A particularly important dose of midostaurin is 200-225 mg/day, in particular 100 mg twice a day (200 mg/day total). The upper limit of dosage is that imposed by side effects and can be determined by trial for the patient being treated.

The terms "imatinib-resistant GIST" or "gastrointestinal stromal tumor are refractory to therapy with imatinib" as used herein define a gastrointestinal stromal tumor for which Imatinib is no longer therapeutically efficient or has a reduced therapeutic effectiveness.

The present invention relates to the use of a combination of imatinib, or a pharmaceutically acceptable salt thereof, and midostaurin, or a pharmaceutically acceptable salt thereof for the preparation of a medicament for the treatment of gastrointestinal stromal tumors, e.g. imatinib-resistant gastrointestinal stromal tumors.

Such a combination therapy is herein referred to as the COMBINATION OF THE INVENTION. The COMBINATION OF THE INVENTION is especially combined preparation. The term "a combined preparation", as used herein defines especially a "kit of parts" in the sense that at least two active ingredients as defined above can be dosed independently or by use of different fixed combinations with distinguished amounts of the ingredients, i.e. simultaneously or at different time points. The parts of the kit can be administered, e.g. simultaneously or chronologically staggered, that is at different time points and with equal or different time intervals for any part of the kit. Preferably, the time intervals are chosen such that the effect on the treated disease in the combined use of the parts is larger than the effect which would be obtained by use of only any one of the active ingredients. The ratio of the total amounts of imatinib to midostaurin to be administered in the combined preparation can be varied, e.g. in order to cope with the needs of a patient sub-population to be treated or the needs of the single patient which different needs can be due to age, sex, body weight, etc...of the patients.

The present invention further relates to packaged imatinib or packaged midostaurin what includes instructions to use both compounds, or salts thereof, together for the treatment of GIST.

In one aspect the present invention provides a treatment of GIST comprising administering a COMBINATION OF THE INVENTION in an amount which is jointly therapeutically effective against GIST to a warm-blooded animal, particularly a human, in need thereof. More particularly, the present invention provides treating a patient suffering from GIST, which comprises administering an effective combination of imatinib, or a pharmaceutically acceptable salt thereof, and midostaurin, or a pharmaceutically acceptable salt thereof, to the patient. More particularly, the present invention provides for treating a patient suffering from GIST, which comprises administering an effective combination of imatinib, or a pharmaceutically acceptable salt thereof, and midostaurin, or a pharmaceutically acceptable salt thereof, to the patient, wherein the imatinib is administered in a dose of from 100 to 1000 mg daily, preferably 200 to 800 mg daily, particularly 400, 600 or 800 mg daily, most particularly 600 mg daily, as an oral pharmaceutical preparation, and the midostaurin is administered in a dose of 100 to 300 mg daily, particularly 150 to 250 mg daily, most particularly 200 mg daily, as an oral pharmaceutical preparation. Most preferably, imatinib is administered as its monomethanesulfonate salt.

The present invention relates to the use of a combination of imatinib, or a pharmaceutically acceptable salt thereof, and midostaurin, or a pharmaceutically acceptable salt thereof for the preparation of a medicament for the treatment of gastrointestinal stromal tumors, e.g. imatinib-resistant gastrointestinal stromal tumors, wherein imatinib and midostaurin are dosed independently. In one embodiment of the invention, imatinib and midostaurin are each administered orally.

### Pharmaceutical Compositions

### Imatinib Pharmaceutical Preparations

Capsules containing 119.5 mg of the imatinib mesylate corresponding to 100 mg of imatinib (free base) as active substance are prepared in the following composition:

**Composition 1**

| | |
|---|---|
| Imatinib mesylate | 119.5 mg |
| Cellulose MK GR | 92 mg |
| Crospovidone XL | 15 mg |
| Aerosil 200 | 2 mg |
| Magnesium stearate | 1.5 mg |
| | 230 mg |

The capsules are prepared by mixing the components and filling the mixture into hard gelatin capsules, size 1.

### Midostaurin Pharmaceutical Preparations

### Composition A:

Gelucire 44/14 (82 parts) is melted by heating to 60° C. Powdered MIDOSTAURIN (18 parts) is added to the molten material. The resulting mixture is homogenised and the dispersion obtained is introduced into hard gelatin capsules of different size, so that some contain a 25mg dosage and others a 75mg dosage of the MIDOSTAURIN. The resulting capsules are suitable for oral administration.

### Composition B:

Gelucire 44/14 (86 parts) is melted by heating to 60° C. Powdered MIDOSTAURIN (14 parts) is added to the molten material. The mixture is homogenised and the dispersion obtained is introduced into hard gelatin capsules of different size, so that some contain a 25mg dosage and others a 75mg dosage of the MIDOSTAURIN. The resulting capsules are suitable for oral administration.

Gelucire 44/14 available commercially from Gattefossé; is a mixture of esters of C8-C18 saturated fatty acids with glycerol and a polyethylene glycol having a molecular weight of about 1500, the specifications for the composition of the fatty acid component being, by weight, 4-10% caprylic acid, 3-9% capric acid, 40-50% lauric acid, 14-24% myristic acid, 4-14% palmitic acid and 5-15% stearic acid.

A preferred example of Gelucire formulation consists of:
Gelucire (44/14): 47 g
MIDOSTAURIN: 3.0g filled into a 60 mL Twist off flask

An example of soft gel will contain the following Microemulsion:

| | |
|---|---|
| Cornoil glycerides | 85.0 mg |
| Polyethylenglykol 400 | 128.25 mg |
| Cremophor RH 40 | 213.75 mg |
| MIDOSTAURIN | 25.0 mg |
| DL alpha Tocopherol | 0.5 mg |
| Ethanol absolute | 33.9 mg |
| Total | 486.4 mg |

### Treatment Example

Adult patients with unresectable or metastatic gastrointestinal stromal tumors (GIST) which are refractory to therapy with imatinib are treated with imatinib mesylate as a single daily dose of 600mg imatinib base and midostaurin at a dose of 100mg twice daily (4 capsules b.i.d.). The morning dose of imatinib should be taken about half an hour before midostaurin. Both drugs are taken with food and with a large glass of water to minimize the risk of GI irritation. In the event that serious side effects occur, the dose of midostaurin is reduced to 100 mg/day (50 mg b.i.d.).

2 out of the 6 patients have stable disease over treatment.

## Claims

1. Use of a combination comprising imatinib or a pharmaceutically acceptable salt thereof and midostaurin or a pharmaceutically acceptable salt thereof for the preparation of a medicament for the treatment of gastrointestinal stromal tumors.

2. The use according to claim 1 wherein imatinib is to be administered in a dose of from 100 to 1000 mg.

3. The use according to claim 2 wherein the dose of imatinib that is to be administered is 200 to 800 mg daily.

4. The use according to claim 3 wherein the dose of imatinib that is to be administered daily is 400, 600 or 800 mg.

5. The use according to claim 1 wherein the midostaurin is to be administered in a dose of 100 to 300 mg daily.

6. The use according to claim 5 wherein the dose is 150 to 250 mg daily.

7. The use according to claim 6 wherein the dose is 200 mg daily.

8. The use according to any one of the preceding claims wherein imatinib and midostaurin are each to be administered orally.

9. The use according to any one of the preceding claims wherein imatinib is to be administered as its monomethanesulfonic acid salt.

10. The use according to any one of the preceding claims wherein imatinib and midostaurin are to be dosed independently.

11. The use according to any one of the preceding claims wherein the gastrointestinal stromal tumor is an imatinib-resistant gastrointestinal stromal tumor.

12. The use of claim 1 wherein gastrointestinal tumors are refractory to therapy with imatinib.

## Patentansprüche

1. Verwendung einer Kombination, die Imatinib oder ein pharmazeutisch akzeptables Salz hiervon und Midostaurin oder ein pharmazeutisch akzeptables Salz hiervon umfasst, zur Herstellung eines Medikaments zur Behandlung von gastrointestinalen stromalen Tumoren.

2. Verwendung nach Anspruch 1, wobei das Imatinib in einer Dosis von 100 bis 1000 mg zu verabreichen ist.

3. Verwendung nach Anspruch 2, wobei die Imatinibdosis, die zu verabreichen ist, 200 bis 800 mg täglich beträgt.

4. Verwendung nach Anspruch 3, wobei die Imatinibdosis, die täglich zu verabreichen ist, 400, 600 oder 800 mg beträgt.

5. Verwendung nach Anspruch 1, wobei das Midostaurin in einer Dosis von 100 bis 300 mg täglich zu verabreichen ist.

6. Verwendung nach Anspruch 5, wobei die Dosis 150 bis 250 mg täglich beträgt.

7. Verwendung nach Anspruch 6, wobei die Dosis 200 mg täglich beträgt.

8. Verwendung nach einem der vorhergehenden Ansprüche, wobei das Imatinib und das Midostarurin jeweils oral zu verabreichen sind.

9. Verwendung nach einem der vorhergehenden Ansprüche, wobei das Imatinib in Form des Monomethansulfonsäuresalzes hiervon zu verabreichen ist.

10. Verwendung nach einem der vorhergehenden Ansprüche, wobei das Imatinib und das Midostaurin unabhängig voneinander zu verabreichen sind.

11. Verwendung nach einem der vorhergehenden Ansprüche, wobei der gastrointestinale stromale Tumor ein Imatinib-resistenter gastrointestinaler stromaler Tumor ist.

12. Verwendung nach Anspruch 1, wobei die gastrointestinalen Tumoren gegenüber einer Therapie mit Imatinib refraktär sind.

## Revendications

1. Utilisation d'une combinaison comprenant de l'imatinib ou un sel pharmaceutiquement acceptable de celui-ci et de la midostaurine ou un sel pharmaceutiquement acceptable de celle-ci, pour la préparation d'un médicament destiné au traitement de tumeurs stromales gastro-intestinales.

2. Utilisation selon la revendication 1, dans laquelle l'imatinib doit être administré à une dose de 100 à 1000 mg.

3. Utilisation selon la revendication 2, dans laquelle la dose d'imatinib devant être administrée est comprise entre 200 et 800 mg par jour.

4. Utilisation selon la revendication 3, dans laquelle la dose d'imatinib devant être administrée quotidiennement est de 400, 600 ou 800 mg.

5. Utilisation selon la revendication 1, dans laquelle la midostaurine doit être administrée à une dose de 100 à 300 mg par jour.

6. Utilisation selon la revendication 5, dans laquelle la dose est comprise entre 150 et 250 mg par jour.

7. Utilisation selon la revendication 6, dans laquelle la dose est de 200 mg par jour.

8. Utilisation selon l'une quelconque des revendications précédentes, dans laquelle l'imatinib et la midostaurine doivent être tous deux administrés par voie orale.

9. Utilisation selon l'une quelconque des revendications précédentes, dans laquelle l'imatinib doit être administré sous la forme de son sel d'acide monométhanesulfonique.

10. Utilisation selon l'une quelconque des revendications précédentes, dans laquelle l'imatinib et la midostaurine doivent être dosés indépendamment.

11. Utilisation selon l'une quelconque des revendications précédentes, dans laquelle la tumeur stromale gastro-intestinale est une tumeur stromale gastro-intestinale résistante à l'imatinib.

12. Utilisation selon la revendication 1, dans laquelle les tumeurs gastro-intestinales sont réfractaires à un traitement par l'imatinib.
